# EUROPÄISCHE PATENTSCHRIFT

(11) **EP 3 765 887 B1**
(45) Veröffentlichungstag und Bekanntmachung des Hinweises auf die Patenterteilung: **01.05.2024**
(21) Anmeldenummer: 19709674.6
(22) Anmeldetag: 05.03.2019
(51) Int. Cl.: G02B 23/24, G02B 5/00, A61B 1/00, A61B 1/05, G02B 7/00, G02B 27/00

(54) **HALTERUNG FÜR EIN OPTISCHES SYSTEM EINES ENDOSKOPS UND VERFAHREN ZUM HERSTELLEN EINER HALTERUNG FÜR EIN OPTISCHES SYSTEM EINES ENDOSKOPS**
HOLDER FOR AN OPTICAL SYSTEM OF AN ENDOSCOPE AND METHOD FOR PRODUCING A HOLDER FOR AN OPTICAL SYSTEM OF AN ENDOSCOPE
SUPPORT POUR UN SYSTÈME OPTIQUE D'UN ENDOSCOPE ET PROCÉDÉ DE FABRICATION D'UN SUPPORT POUR UN SYSTÈME OPTIQUE D'UN ENDOSCOPE

(30) Priorität: 14.03.2018 DE 102018105845
(43) Veröffentlichungstag der Anmeldung: 20.01.2021
(73) Patentinhaber: OLYMPUS Winter & Ibe GmbH, 22045 Hamburg (DE)
(72) Erfinder: SCHÖLER, Uwe, 22955 Hoisdorf (DE); TORKUHL, Nils, 22885 Barsbüttel (DE); WIETERS, Martin, 22885 Barsbüttel (DE)
(74) Vertreter: Seemann & Partner Patentanwälte mbB
(86) Internationale Anmeldenummer: PCT/EP2019/055404
(87) Internationale Veröffentlichungsnummer: WO 2019/174966

(56) Entgegenhaltungen:
- EP-A1- 1 455 216
- WO-A1-2018/024548
- US-B1- 6 767 322

## Beschreibung

Die Erfindung betrifft eine Halterung für ein optisches System eines Endoskops umfassend einen Aufnahmebereich, der zur Aufnahme eines optischen Elements eingerichtet ist, und wenigstens einen Fixierbereich, der zur Fixierung der Halterung im optischen System mittels einer Lötverbindung eingerichtet und separat vom Aufnahmebereich ist. Außerdem betrifft die Erfindung ein optisches System für ein Endoskop, ein Endoskop und ein Verfahren zum Herstellen einer Halterung für ein optisches System eines Endoskops.

Bei medizinischen Endoskopen ist ein geringer Außendurchmesser des Endoskopschafts wünschenswert. Durch die zunehmende Miniaturisierung stellt die Herstellung und Montage des optischen Systems eine technische Herausforderung dar. So muss beispielsweise bei der Miniaturisierung des optischen Systems gewährleistet sein, dass die optischen Eigenschaften des Endoskops den Vorgaben entsprechen und das Endoskop gleichzeitig den Belastungen standhält, denen es im Betrieb und bei der Aufbereitung ausgesetzt wird. Dies gilt vor allem auch für die Halterungen im optischen System, die Komponenten wie beispielsweise optische Elemente und Bildsensoren aufnehmen.

Stereo-Videoendoskope verfügen über zwei voneinander getrennte Linsensystemkanäle, die jeweils Lichtbündel aus einem Blickfeld mit leicht unterschiedlichem Blickwinkel auf jeweils einen Bildsensor abbilden. Auf diese Weise lässt sich ein Stereobild des Aufnahmebereichs zusammensetzen, das einem Betrachter einen räumlichen Eindruck vermittelt. Die Verwendung von zwei Linsensystemkanälen erfordert eine besonders platzsparende Ausführung des optischen Systems, um den Außendurchmesser des Stereo-Videoendoskops nicht unnötig zu vergrößern.

Aus US 6,767,322 B1 ist eine Halterung für ein optisches System eines Endoskops bekannt, welche einen Aufnahmebereich umfasst, der zur Aufnahme eines optischen Elements eingerichtet ist. Die Halterung umfasst wenigstens einen Fixierbereich, der zur Fixierung der Halterung im optischen System mittels einer Lötverbindung eingerichtet ist. Die Halterung ist hergestellt aus einem Grundmaterial, das mit einer Beschichtung versehen ist. Das Material der Beschichtung weist eine bessere Lötbarkeit als das Grundmaterial auf.

WO 2018/024548 A1 zeigt ein optisches System eines Stereo-Videoendoskops mit seitlicher Blickrichtung. Eine Innenseite eines das optische System aufnehmenden Tubus ist geschwärzt, um Lichtstrahlen zu absorbieren, die von außerhalb des Blickfelds einfallen.

Es ist eine Aufgabe der Erfindung, eine Halterung für ein optisches System eines Endoskops, ein optisches System für ein Endoskop, ein Endoskop und ein Verfahren zum Herstellen einer Halterung für ein optisches System eines Endoskops anzugeben, wobei die Halterung einen stabilen und zuverlässigen Aufbau bei kleinem Bauraum ermöglichen soll.

Diese Aufgabe wird gelöst durch eine Halterung für ein optisches System eines Endoskops umfassend einen Aufnahmebereich, der zur Aufnahme eines optischen Elements eingerichtet ist, und wenigstens einen Fixierbereich, der zur Fixierung der Halterung im optischen System mittels einer Lötverbindung eingerichtet und separat vom Aufnahmebereich ist, wobei das Material der Halterung ein Grundmaterial ist, das mit einer Beschichtung versehen ist, wobei das Material der Beschichtung eine bessere Lötbarkeit als das Grundmaterial aufweist, wobei die Halterung dadurch weitergebildet ist, dass mittels Laseroberflächenbearbeitung die Beschichtung im Aufnahmebereich abgetragen und die Oberfläche des Aufnahmebereichs mittels der Laseroberflächenbearbeitung geschwärzt ist.

Im Kontext der vorliegenden Beschreibung wird unter einer distalen optischen Baugruppe eine optische Baugruppe verstanden, die distal der ebenfalls erwähnten proximalen optischen Baugruppe liegt. Gleiches gilt für die proximale optische Baugruppe.

Durch die erfindungsgemäße Halterung kann zuverlässig im optischen System verlötet werden, da die Halterung im Fixierbereich mit einer gut lötbaren Beschichtung versehen ist. Andererseits wird ein Verlust der Qualität der Abbildungseigenschaften durch Reflexionen einfallender Lichtbündel im Aufnahmebereich des optischen Elements vermieden, indem dieser Aufnahmebereich geschwärzt ist.

Halterungen im optischen System eines Endoskops müssen präzise gefertigt sein und eine hohe Haltbarkeit aufweisen. Zudem darf sich der Wärmeausdehnungskoeffizient des Materials der Halterung nicht zu stark vom Wärmeausdehnungskoeffizienten der Materialien eines aufgenommenen optischen Elements, also beispielsweise Glas, Silizium und Keramik, unterscheiden. Das Grundmaterial ist ein Material der Halterung, welches sich durch Einwirkung eines Laserstrahls schwärzen lässt. Es handelt sich bei dem Grundmaterial insbesondere um ein Metall, beispielsweise eine Stahllegierung.

Als Material für die Beschichtung wird vorzugsweise ein Material mit einer guten Lötbarkeit verwendet, wie beispielsweise Gold, Silber, Zinn oder Palladium. Da diese Materialien eine deutlich bessere Lötbarkeit aufweisen als beispielsweise eine Stahllegierung, kann der Fixierbereich der Halterung mittels einer Lötverbindung zuverlässig und stabil mit einem entsprechenden Fixierelement des optischen Systems verbunden werden. Die Beschichtung wird beispielsweise galvanisch aufgetragen. Es sind jedoch ebenso andere Arten der Beschichtung, mit denen eine dünne Schicht aufgetragen werden kann, vorgesehen. Beispielsweise kann die Beschichtung aufgedampft werden. Beim Beschichtungsprozess wird das für die Beschichtung eingesetzte Material vielfach auf einen Großteil der gesamten Oberfläche der Halterung aufgetragen. Die für die Beschichtung verwendeten Materialien haben jedoch zumindest in Teilen des Wellenlängenbereichs des sichtbaren Lichts vielfach einen hohen Reflexionsgrad. Dies führt dazu, dass es im mit großer Wahrscheinlichkeit anfänglich ebenfalls beschichteten Aufnahmebereich zu Reflexionen von einfallenden Lichtbündeln an den beschichteten Oberflächen kommen kann. Dies würde die optische Qualität des optischen Systems verringern.

Um diesen unerwünschten Effekt zu vermeiden, könnte der Versuch unternommen werden, beispielsweise nur den Fixierbereich des Halters zu beschichten. Eine solche gezielte Teilbeschichtung nur des Fixierbereichs ist jedoch technisch aufwändig. Gemäß der Erfindung wird daher eine anfängliche Beschichtung des Aufnahmebereichs in Kauf genommen und die an dieser Stelle unerwünschte Beschichtung nachträglich mittels einer Laseroberflächenbearbeitung wieder abgetragen.

Außerdem wird mittels der durchgeführten Laseroberflächenbearbeitung die Oberfläche des Halters im Aufnahmebereich geschwärzt. So können vorteilhaft Reflexionen im Aufnahmebereich, im Vergleich zu einer unbehandelten Oberfläche des Grundmaterials, weiter reduziert werden. Bei einer solchen Laseroberflächenbearbeitung lässt sich der bearbeitete Bereich sehr präzise begrenzen, so dass vorteilhaft nur der Aufnahmebereich bearbeitet wird.

Unter einer Laseroberflächenbearbeitung wird im Kontext der vorliegenden Beschreibung eine Bearbeitung einer Oberfläche eines Materials mittels eines Laserstrahls verstanden, wie sie beispielsweise von Laserablation und der Laserbeschriftung bekannt ist. Der Begriff "Laseroberflächenbearbeitung" schließt also sowohl das Abtragen von Material (Laserablation) als auch die Veränderung der Oberfläche wie bei der Laserbeschriftung mit dem Ziel der Schwärzung ein.

Das Grundmaterial aus dem der Halter hergestellt ist, ist bevorzugt eine Stahllegierung, insbesondere Chromstahl. Vorteilhaft weist Chromstahl die erforderliche Haltbarkeit, insbesondere die gewünschte Duktilität auf und lässt sich außerdem mittels Laseroberflächenbearbeitung gut und effizient schwärzen. Bevorzugt wird ein Chromstahl mit der Werkstoffnummer 1.4104 oder 1.4021 eingesetzt. Es können jedoch auch andere Stahllegierungen oder andere Materialien eingesetzt werden, die sich mittels Laseroberflächenbearbeitung schwärzen lassen.

Bevorzugt ist mittels Laseroberflächenbearbeitung mit einem Pikosekundenlaser oder einem Femtosekundenlaser die Beschichtung im Aufnahmebereich abgetragen und auch die Oberfläche des Aufnahmebereichs geschwärzt. Ein Ultrakurzpulslaser ist beispielsweise ein Pikosekundenlaser oder ein Femtosekundenlaser. Der Einsatz eines Ultrakurzpulslasers ist vorteilhaft, da die Beschichtung effizient abgetragen und auch die Oberfläche effizient geschwärzt werden kann.

Gemäß einer weiteren Ausführungsform ist das optische Element, zu dessen Aufnahme der Aufnahmebereich eingerichtet ist, ein optisches Umlenkelement, nämlich ein Umlenkprisma. Mittels eines optischen Umlenkelements lässt sich die Ausbreitungsrichtung eines in das optische Umlenkelement einfallenden Strahlenbündels verändern. Ein Umlenkprisma ist als ein solches optisches Umlenkelement vorgesehen. Durch Schwärzen des Aufnahmebereichs lassen sich Reflexionen am Aufnahmebereich des optischen Umlenkelements reduzieren oder verhindern, die die Bildqualität des Endoskops verschlechtern würden.

Gemäß einer weiteren Ausführungsform ist die Halterung dazu eingerichtet, einen Bildsensor derart aufzunehmen, dass sich eine lichtempfindliche Sensorfläche des aufgenommenen Bildsensors parallel zu einer Einfallsrichtung erstreckt, wobei mittels des optischen Umlenkprismas entlang der Einfallsrichtung in das optische Umlenkprisma einfallende Lichtbündel in Richtung des aufgenommenen Bildsensors umgelenkt werden.

Die Einfallsrichtung entspricht vorzugsweise einer optischen Achse des optischen Systems. Durch Aufnahme eines Bildsensors, dessen aktive Fläche sich parallel zur Einfallsrichtung erstreckt, wird eine raumsparende Anordnung des Bildsensors im optischen System erreicht. Die Halterung ist gemäß dieser Ausführungsform also dazu ausgebildet, sowohl den Bildsensor als auch das optische Umlenkprisma aufzunehmen.

Die Aufgabe wird außerdem gelöst durch ein optisches System für ein Endoskop, umfassend wenigstens eine Halterung gemäß einer oder mehrerer der zuvor beschriebenen Ausführungsformen und wenigstens ein optisches Element, wobei das optische Element im Aufnahmebereich der Halterung aufgenommen und die Halterung mittels wenigstens einer ersten oder zweiten Lötverbindung im optischen System fixiert ist, wobei die erste oder zweite Lötverbindung den Fixierbereich der Halterung mit einem ersten oder zweiten Fixierelement des optischen Systems verbindet.

Vorzugsweise wird das optische Element im Aufnahmebereich der Halterung mittels eines Klebeverfahrens fixiert.

Auf das optische System treffen gleiche oder ähnliche Vorteile zu, wie sie bereits im Hinblick auf die Halterung erwähnt wurden, so dass auf Wiederholungen verzichtet werden soll. Es wird vorteilhaft ein kompaktes, stabiles und hochwertiges optisches System angegeben.

Bevorzugt weist die Halterung einen ersten Fixierbereich und einen zweiten Fixierbereich auf, wobei der erste Fixierbereich mittels der ersten Lötverbindung mit dem ersten Fixierelement und der zweite Fixierbereich mittels der zweiten Lötverbindung mit dem zweiten Fixierelement verbunden sind. Die Halterung ist also mittels zwei Lötverbindungen im optischen System fixiert. Vorteilhaft wird dadurch die Stabilität des optischen Systems erhöht.

Gemäß einer Ausführungsform ist das optische System zum Einsatz in einem Stereo-Videoendoskop ausgebildet, wobei das optische System einen ersten Linsensystemkanal mit einer ersten optischen Achse und einen zweiten Linsensystemkanal mit einer zweiten optischen Achse umfasst, wobei die erste optische Achse parallel zur zweiten optischen Achse verläuft, wobei die wenigstens eine Halterung des optischen Systems eine erste Halterung und eine zweite Halterung umfasst und das wenigstens eine optische Element des optischen Systems ein erstes optisches Element und ein zweites optisches Element umfasst, wobei im Aufnahmebereich der ersten Halterung das erste optische Element und im Aufnahmebereich der zweiten Halterung das zweite optische Element aufgenommen sind, wobei das optische System derart eingerichtet ist, dass entlang des ersten Linsensystemkanals geführte Lichtbündel von dem ersten optischen Element abgelenkt und entlang des zweiten Linsensystemkanals geführte Lichtbündel von dem zweiten optischen Element abgelenkt werden. Gemäß dieser Ausführungsform ist somit für jeden Linsensystemkanal des optischen Systems eine Halterung mit einem optischen Element vorgesehen.

Das optische System ist bevorzugt ein optisches System eines Stereo-Videoendoskops. Die Begriffe der distalen optischen Baugruppe und der proximalen optischen Baugruppe werden im Kontext der vorliegenden Beschreibung und im Kontext eines Stereo-Videoendoskops so verstanden, dass sowohl die distale als auch die proximale optische Baugruppe ein Teil einer Baugruppe sind, in der die beiden optischen Kanäle (linker Kanal und rechter Kanal) separat geführt werden.

Durch die erfindungsgemäßen Halterungen wird ein besonders raumsparender und stabiler Aufbau bei geringem Fertigungsaufwand und guten optischen Eigenschaften realisiert. Die erste und die zweite Halterung sind jeweils so ausgestaltet, wie es in Bezug auf die Halterung gemäß Aspekten der Erfindung oben beschrieben ist. Die erste und die zweite Halterung sind also insbesondere konstruktiv identisch aufgebaut.

Bevorzugt sind die erste Halterung und die zweite Halterung symmetrisch zueinander im optischen System angeordnet, wobei insbesondere die erste Halterung achsensymmetrisch zur zweiten Halterung in Bezug auf eine Längsachse des optischen Systems angeordnet ist, wobei die Längsachse mittig zwischen der ersten optischen Achse und der zweiten optischen Achse verläuft. Durch die symmetrische Anordnung der Halterungen wird das optische System noch raumsparender und haltbarer.

Gemäß einer weiteren Ausführungsform ist jede Halterung jeweils zur Aufnahme eines Bildsensors ausgebildet, wobei die aktiven Flächen der Bildsensoren jeweils parallel zu den Linsensystemkanälen angeordnet sind, und wobei das erste optische Element und das zweite optische Element optische Umlenkelemente, insbesondere Umlenkprismen, sind, die dazu eingerichtet sind, in die optischen Umlenkelemente einfallende Lichtbündel in Richtung der Bildsensoren umzulenken.

Die Aufgabe wird außerdem gelöst durch ein Endoskop, insbesondere Stereo-Videoendoskop, umfassend ein optisches System gemäß einer der zuvor beschriebenen Ausführungsformen.

Auch auf das Endoskop treffen gleiche oder ähnliche Vorteile zu, wie sie bereits oben im Hinblick auf die Halterung und das optische System erwähnt wurden, so dass auf Wiederholungen verzichtet werden soll.

Die Aufgabe wird ferner gelöst durch ein Verfahren zum Herstellen einer Halterung für ein optisches System eines Endoskops mit den folgenden, nacheinander auszuführenden Verfahrensschritten:
- Bereitstellen eines Rohlings der Halterung aus einem Grundmaterial,
- Beschichten des Rohlings mit einer Beschichtung, wobei die Beschichtung eine bessere Lötbarkeit als das Grundmaterial aufweist, wobei auf dem beschichteten Rohling ein Fixierbereich zum Fixieren im optischen System mittels einer Lötverbindung vorgesehen wird, gekennzeichnet durch den folgenden Verfahrensschritt:
- Bearbeiten eines Aufnahmebereichs des beschichteten Rohlings mit einem Laser, wobei der Aufnahmebereich zur Aufnahme eines optischen Elements eingerichtet und separat vom Fixierbereich ist, und wobei durch die Laserbearbeitung die Beschichtung im Aufnahmebereich abgetragen und die Oberfläche des Aufnahmebereichs geschwärzt wird.

Vorteilhaft wird ein präzises und effizientes Verfahren zum Herstellen einer Halterung für ein optisches System eines Endoskops bereitgestellt.

Bevorzugt erfolgt die Laserbearbeitung des Aufnahmebereichs mit einem Pikosekundenlaser oder einem Femtosekundenlaser.

Weiterhin bevorzugt erfolgen das Abtragen der Beschichtung im Aufnahmebereich und das Schwärzen des Aufnahmebereichs mit einer einzigen Laserquelle, wobei insbesondere das Abtragen der Beschichtung im Aufnahmebereich und das Schwärzen des Aufnahmebereichs in einem einzigen Arbeitsschritt durchgeführt werden. Vorteilhaft werden dadurch der Aufwand bei der Herstellung und die Herstellungskosten reduziert.

Schließlich wird die Aufgabe gelöst durch eine nicht beanspruchte Halterung, die sich dadurch auszeichnet, dass sie mit dem zuvor beschriebenen Verfahren hergestellt ist. Auf die Halterung treffen die oben bereits genannten Vorteile in gleicher oder ähnlicher Weise zu.

Weitere Merkmale der Erfindung werden aus der Beschreibung erfindungsgemäßer Ausführungsformen zusammen mit den Ansprüchen und den beigefügten Zeichnungen ersichtlich.

### Die Erfindung wird nachstehend

anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische abschnittsweise Darstellung eines Endoskops,
- Fig. 2: eine schematisch vereinfachte Darstellung eines optischen Systems für ein Stereo-Videoendoskop mit zwei Halterungen,
- Fig. 3: eine schematisch vereinfachte Darstellung einer distalen Baugruppe eines optischen Systems für ein Stereo-Videoendoskop mit zwei Fixierelementen,
- Fig. 4: eine schematisch vereinfachte Darstellung eines optischen Systems für ein Stereo-Videoendoskop mit zwei Halterungen in einer gegenüber der Darstellung in Fig. 3 gedrehten Ansicht,
- Fig. 5: eine schematisch vereinfachte perspektivische Darstellung einer Halterung für ein optisches System eines Endoskops,
- Fig. 6a bis 6c: eine schematische Darstellung eines Verfahrens zum Herstellen einer Halterung für ein optisches System eines Endoskops,
- Fig. 7: eine schematisch vereinfachte perspektivische Darstellung einer Halterung für ein optisches System eines Endoskops mit einem Umlenkprisma und einem Bildsensor und
- Fig. 8: eine schematisch vereinfachte perspektivische Darstellung einer Halterung für ein optisches System eines Endoskops mit einem Umlenkprisma und einem Bildsensor in einer im Vergleich zu der Darstellung in Fig. 7 um die Längsachse des Halters gedrehten Ansicht.

In den Zeichnungen sind jeweils gleiche oder gleichartige Elemente und/oder Teile mit denselben Bezugsziffern versehen, so dass von einer erneuten Vorstellung jeweils abgesehen wird.

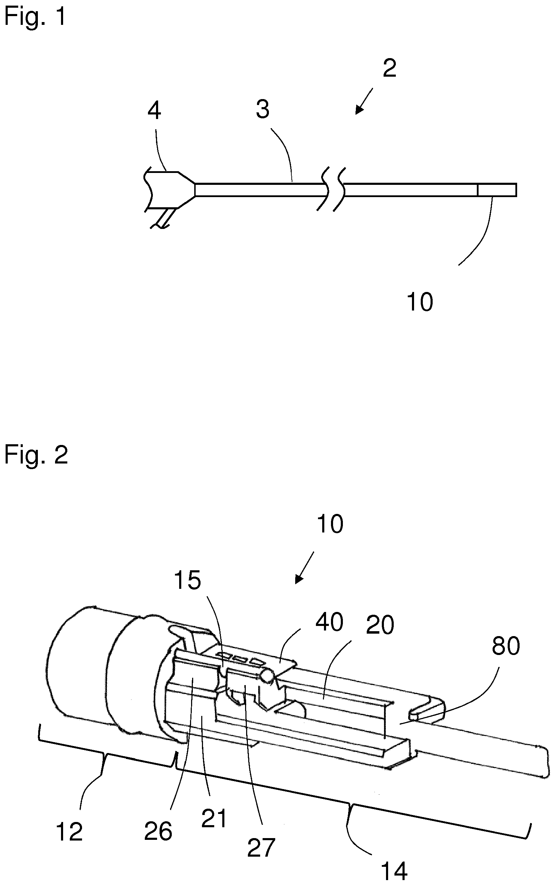

Fig. 1 zeigt schematisch und vereinfacht ein abschnittsweise gezeigtes Endoskop 2 mit einem Schaft 3 und einem Handgriff 4. Das Endoskop 2 ist beispielsweise ein Videoendoskop, ferner beispielsweise ein Stereo-Videoendoskop. An einem distalen Ende des Schafts 3 befindet sich ein optisches System 10.

Ein solches optisches System 10 für ein Stereo-Videoendoskop ist in Fig. 2 in einer schematisch vereinfachten perspektivischen Darstellung gezeigt. Das optische System 10 umfasst eine distale optische Baugruppe 12 und eine proximale optische Baugruppe 14. Das optische System 10, also die distale optischen Baugruppe 12 und die proximale optische Baugruppe 14, befindet sich in einem Teil oder Abschnitt des gesamten optischen Systems des Stereo-Videoendoskops, in dem die beiden optischen Kanäle (linker Kanal und rechter Kanal) separat geführt werden.

In der distalen optischen Baugruppe 12 ist eine Anzahl von in Fig. 2 nicht dargestellten optischen Elementen angeordnet. Aus einem Behandlungs- und/oder Beobachtungsraum an einer distalen Spitze des Schafts 3 einfallende Lichtbündel werden von der distalen optischen Baugruppe 12 empfangen und an die proximale optische Baugruppe 14 weitergeleitet. Die proximale optische Baugruppe 14 umfasst zwei Bildsensoren 40, von denen in Fig. 2 lediglich einer sichtbar ist. Die Bildsensoren 40 sind mit (beispielsweise flexiblen) Leiterplatten 80 zur Weiterleitung von Bildsignalen in Richtung des Handgriffs 4 gekoppelt, beispielsweise auf den betreffenden Leiterplatten 80 montiert.

Die in Fig. 2 gezeigte Ausführungsform des optischen Systems 10 umfasst eine proximale optische Baugruppe 14 mit einer ersten Halterung 20 und einer zweiten Halterung 21, die zur Aufnahme der Bildsensoren 40 ausgebildet sind. Um die proximale optische Baugruppe 14 an der distalen optischen Baugruppe 12 zu fixieren, weisen die erste Halterung 20 und die zweite Halterung 21 jeweils einen ersten Fixierbereich 26 und einen zweiten Fixierbereich 27 auf. Die Fixierbereiche 26, 27 sind zur Aufnahme eines zylinderförmigen Fixierelements 15 ausgebildet.

In der in Fig. 2 gewählten Darstellung ist lediglich der erste Fixierbereich 26 der zweiten Halterung 21 und der zweite Fixierbereich 27 der ersten Halterung 20 sichtbar, die mit einem ersten Fixierelement 15 verlötet sind. Das zweite Fixierelement 16 ist in Fig. 2 verdeckt. Die Fixierelemente 15, 16 werden an der distalen optischen Baugruppe 12 befestigt und jeweils am ersten Fixierbereich 26 einer Halterung 20, 21 und am zweiten Fixierbereich 27 der anderen Halterung 20, 21 mit einer Lötverbindung fixiert. Auf diese Weise wird eine sehr stabile und platzsparende Anordnung der Halterungen 20, 21 erreicht.

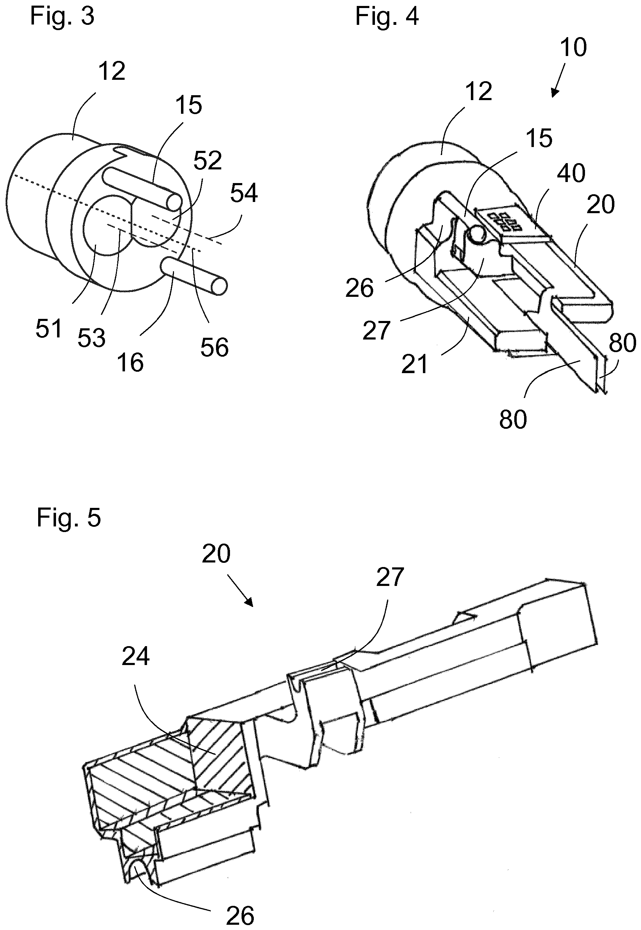

Fig. 3 zeigt eine schematisch vereinfachte Darstellung der distalen optischen Baugruppe 12, in der die beiden Fixierelemente 15, 16 zu erkennen sind. Zudem sind ein erster Linsensystemkanal 51 mit einer ersten optischen Achse 53 und ein zweiter Linsensystemkanal 52 mit einer zweiten optischen Achse 54 dargestellt. Eine Längsachse 56 der optischen Baugruppe 12 erstreckt sich mittig zwischen den beiden optischen Achsen 53, 54. Dabei ist das erste Fixierelement 15 oberhalb und das zweite Fixierelement 16 unterhalb der Linsensystemkanäle 51, 52 angeordnet.

In Fig. 4 ist eine weitere schematisch vereinfachte perspektivische Darstellung des optischen Systems 10 gezeigt. In dieser Darstellung ist zu erkennen, dass jeweils eine Leiterplatte 80 mit einem Bildsensor 40 verbunden ist. Da die Bildsensoren 40 parallel zur Längsachse 56 angeordnet sind, sind in den Halterungen 20, 21 Umlenkprismen angeordnet, die in den Fig. 2 bis 4 verdeckt sind. Die Umlenkprismen lenken die entlang der optischen Achsen 53, 54 einfallenden Lichtbündel in Richtung der Bildsensoren 40 um.

Fig. 5 zeigt eine schematisch vereinfachte perspektivische Darstellung einer Halterung 20. In dieser Darstellung sind die Fixierbereiche 26, 27 zu erkennen, mit denen die Halterung 20 im optischen System 10 an den Fixierelementen 15, 16 fixiert wird. Weiterhin zeigt Fig. 5 den schraffiert dargestellten Aufnahmebereich 24, der zur Aufnahme eines optischen Elements ausgebildet ist. Werden die Bildsensoren 40 parallel zur Längsachse 56 angeordnet, wie beispielsweise in den Fig. 2 bis 4 gezeigt, wird als optisches Element beispielsweise ein Umlenkprisma verwendet, welches entlang der optischen Achsen 53, 54 einfallende Lichtbündel in Richtung der Bildsensoren 40 umlenkt.

Um die Stabilität der Halterung 20 zu gewährleisten, muss diese aus einem stabilen und duktilen Material gefertigt sein, dass außerdem einen ähnlichen Wärmeausdehnungskoeffizienten aufweist wie die Materialien des optischen Elements. Damit die Lötverbindung zwischen den Fixierbereichen 26, 27 und den Fixierelementen 15, 16 haltbar ist, müssen die Fixierbereiche 26, 27 zudem eine hohe Lötbarkeit aufweisen. Schließlich sollen im Aufnahmebereich 24 keine Reflexionen auftreten, da diese sich negativ auf die Bildqualität des Endoskops 2 auswirken.

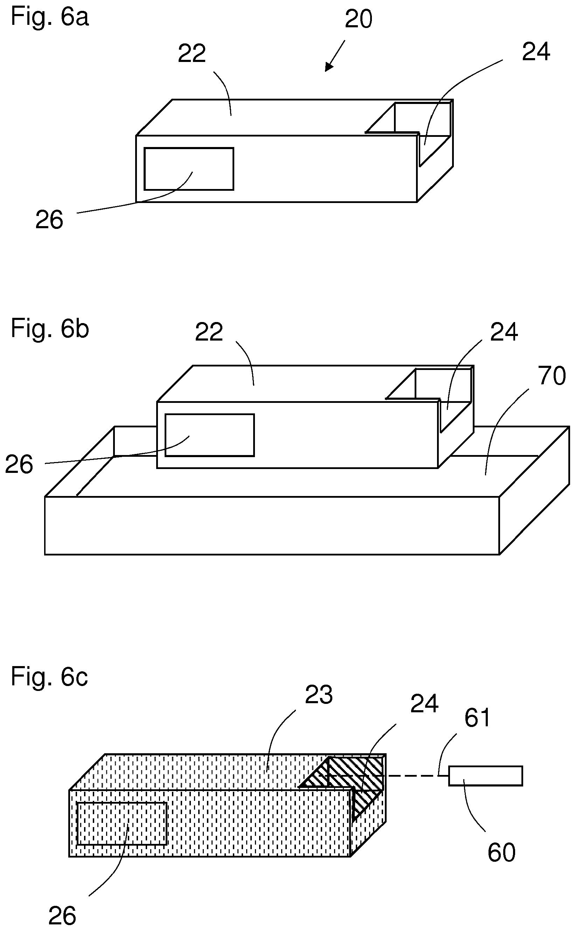

Um all diesen Anforderungen zu genügen, wird zur Herstellung der Halterungen 20, 21 ein Verfahren eingesetzt, das in den Fig. 6a bis 6c schematisch beschrieben ist. Wie in Fig. 6a schematisch gezeigt, wird zunächst ein Rohling 2 der Halterung 20 aus einem Grundmaterial, beispielsweise einer Stahllegierung, ferner beispielsweise Chromstahl bereitgestellt oder zuvor gefertigt. Abweichend von den vorherigen Darstellungen wird der Rohling schematisch stark vereinfacht dargestellt, da lediglich die durchgeführten Verfahrensschritte, nicht jedoch dessen konstruktive Ausgestaltung erläutert werden soll.

Der Rohling 22 weist wenigstens einen Fixierbereich 26 und einen Aufnahmebereich 24 auf. Da ein Chromstahl verwendet wird, der eine relativ schlechte Lötbarkeit aufweist, ist der Fixierbereich 26 jedoch noch nicht zur Fixierung mittels einer Lötverbindung geeignet.

In Fig. 6b ist gezeigt, wie der Rohling 22 mit einer Beschichtung 23, z. B. einer Goldschicht, versehen wird. Dies geschieht beispielsweise, wie in Fig. 6b angedeutet, in einem galvanischen Bad 70. In diesem Prozess wird ein Großteil der Oberfläche des Rohlings 22 und damit auch der Fixierbereich 26 mit der Beschichtung 23 beschichtet. Durch die Beschichtung 23 weist der Fixierbereich 26 eine stark verbesserte Lötbarkeit auf.

Nach dem Beschichten wird der Aufnahmebereich 24 mit einem Ultrakurzpulslaser bearbeitet, wie in Fig. 6c angedeutet. Durch die Bearbeitung mit dem Laserstrahl 61 wird die Beschichtung 23 abgetragen und der darunterliegende Chromstahl im Aufnahmebereich 24 geschwärzt, so dass Reflexionen in diesem Bereich vermieden werden. Die Beschichtung 23 der Grundform 22 ist dabei mit einer vertikalen Schraffur und der geschwärzte Aufnahmebereich 24 mit einer durchgezogenen quer verlaufenden Schraffur angedeutet.

Im Gegensatz zum Verfahrensschritt des Beschichtens ist die Laseroberflächenbearbeitung bezüglich der bearbeiteten Fläche sehr präzise, so dass das Abtragen und Schwärzen präzise auf den Aufnahmebereich 24 beschränkt wird. Um bei der Laseroberflächenbearbeitung Kosten und Zeit zu sparen, werden sowohl das Abtragen als auch das Schwärzen mit einer einzigen Laserquelle 60 durchgeführt. Vorteilhafterweise erfolgen beide Vorgänge sogar in einem einzigen Arbeitsschritt.

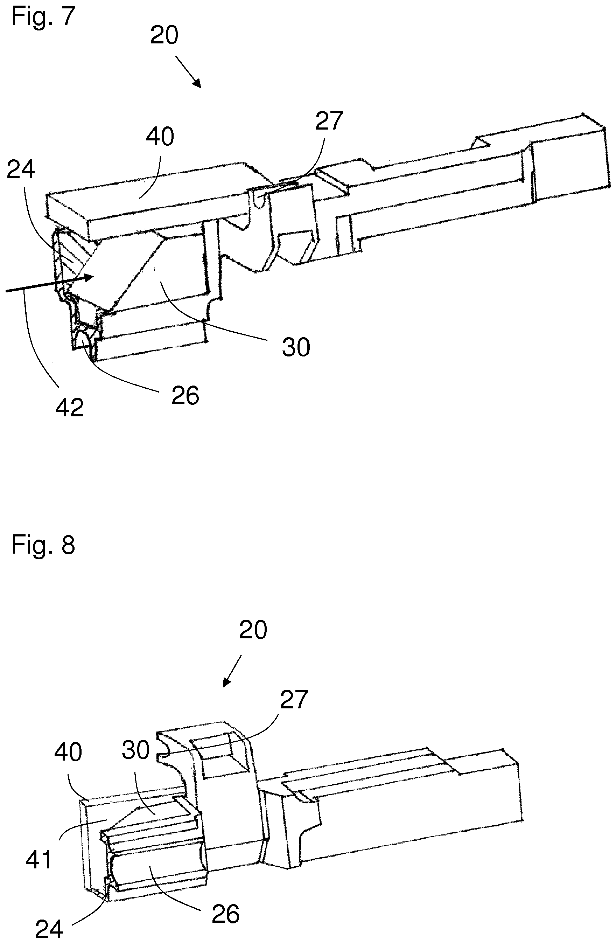

In Fig. 7 und 8 sind schematisch und vereinfacht zwei verschiedene perspektivische Ansichten der Halterung aus Fig. 5 nach erfolgter Bearbeitung dargestellt. Ein optisches Element 30, beispielhaft ein Umlenkprisma, ist in den geschwärzten Aufnahmebereich 24 eingesetzt und dort fixiert, beispielsweise eingeklebt, worden. Zudem ist ein Bildsensor 40 so auf der Halterung 20 angeordnet, dass aus der Einfallsrichtung 42 einfallende Lichtbündel von dem Umlenkprisma in Richtung einer aktiven Fläche 41 des Bildsensors 40 abgelenkt werden. Die Einfallsrichtung 42 entspricht im fixierten Zustand der Halterung 20 einer der optischen Achsen 53, 54 des optischen Systems 10.

### Bezugszeichenliste

- 2: Endoskop
- 3: Schaft
- 4: Handgriff
- 10: optisches System
- 12: distale optische Baugruppe
- 14: proximale optische Baugruppe
- 15: erstes Fixierelement
- 16: zweites Fixierelement
- 20: erste Halterung
- 21: zweite Halterung
- 22: Rohling
- 23: Beschichtung
- 24: Aufnahmebereich
- 26: erster Fixierbereich
- 27: zweiter Fixierbereich
- 30: optisches Element
- 40: Bildsensor
- 41: optisch aktive Fläche
- 42: Einfallsrichtung
- 51: erster Linsensystemkanal
- 52: zweiter Linsensystemkanal
- 53: erste optische Achse
- 54: zweite optische Achse
- 56: Längsachse
- 60: Laserquelle
- 61: Laserstrahl
- 70: galvanisches Bad
- 80: Leiterplatte

## Patentansprüche

1. Halterung (20, 21) für ein optisches System (10) eines Endoskops (2), umfassend einen Aufnahmebereich (24), der zur Aufnahme eines optischen Elements (30) eingerichtet ist, und wenigstens einen Fixierbereich (26, 27), der zur Fixierung der Halterung (20, 21) im optischen System (10) mittels einer Lötverbindung eingerichtet und separat vom Aufnahmebereich (24) ist, wobei das Material der Halterung (20, 21) ein Grundmaterial ist, das mit einer Beschichtung (23) versehen ist, wobei ein Material der Beschichtung (23) eine bessere Lötbarkeit als das Grundmaterial aufweist, **dadurch gekennzeichnet, dass** mittels Laseroberflächenbearbeitung die Beschichtung (23) im Aufnahmebereich (24) abgetragen und die Oberfläche des Aufnahmebereichs (24) mittels der Laseroberflächenbearbeitung geschwärzt ist.

2. Halterung (20, 21) nach Anspruch 1, **dadurch gekennzeichnet, dass** das Grundmaterial eine Stahllegierung, insbesondere ein Chromstahl ist.

3. Halterung (20, 21) nach Anspruch 1 oder 2, **dadurch gekennzeichnet, dass** mittels Laseroberflächenbearbeitung mit einem Pikosekundenlaser oder einem Femtosekundenlaser die Beschichtung (23) im Aufnahmebereich (24) abgetragen und mittels der Laseroberflächenbearbeitung mit dem Pikosekundenlaser oder dem Femtosekundenlaser die Oberfläche des Aufnahmebereichs (24) geschwärzt ist.

4. Halterung (20, 21) nach einem der Ansprüche 1 bis 3, **dadurch gekennzeichnet, dass** das optische Element (30), zu dessen Aufnahme der Aufnahmebereich (24) eingerichtet ist, ein optisches Umlenkprisma ist.

5. Halterung (20, 21) nach Anspruch 4, **dadurch gekennzeichnet, dass** die Halterung (20, 21) dazu eingerichtet ist, einen Bildsensor (40) derart aufzunehmen, dass sich eine lichtempfindliche Sensorfläche (41) des aufgenommenen Bildsensors (40) parallel zu einer Einfallsrichtung (42) erstreckt, wobei mittels des optischen Umlenkprismas entlang der Einfallsrichtung (42) in das optische Umlenkprisma einfallende Lichtbündel in Richtung des aufgenommenen Bildsensors (40) umgelenkt werden.

6. Optisches System (10) für ein Endoskop (2), umfassend wenigstens eine Halterung (20, 21) nach einem der Ansprüche 1 bis 5 und wenigstens ein optisches Element (30), wobei das optische Element (30) im Aufnahmebereich (24) der Halterung (20, 21) aufgenommen und die Halterung (20, 21) mittels wenigstens einer ersten oder zweiten Lötverbindung im optischen System (10) fixiert ist, wobei die erste oder zweite Lötverbindung den Fixierbereich (26, 27) der Halterung (20, 21) mit einem ersten oder zweiten Fixierelement (15, 16) des optischen Systems (10) verbindet.

7. Optisches System (10) nach Anspruch 6, **dadurch gekennzeichnet, dass** die Halterung (20, 21) einen ersten Fixierbereich (26) und einen zweiten Fixierbereich (27) aufweist, wobei der erste Fixierbereich (26) mittels der ersten Lötverbindung mit dem ersten Fixierelement (15) und der zweite Fixierbereich (27) mittels der zweiten Lötverbindung mit dem zweiten Fixierelement (16) verbunden sind.

8. Optisches System (10) nach Anspruch 6 oder 7, **dadurch gekennzeichnet, dass** das optische System (10) zum Einsatz in einem Stereo-Videoendoskop ausgebildet ist, wobei das optische System (10) einen ersten Linsensystemkanal (51) mit einer ersten optischen Achse (53) und einen zweiten Linsensystemkanal (52) mit einer zweiten optischen Achse (54) umfasst, wobei die erste optische Achse (53) parallel zur zweiten optischen Achse (54) verläuft, und wobei die wenigstens eine Halterung (20, 21) des optischen Systems (10) eine erste Halterung (20) nach einem der Ansprüche 1 bis 5 und eine zweite Halterung (21) nach einem der Ansprüche 1 bis 5 umfasst und das wenigstens eine optische Element (30) des optischen Systems (10) ein erstes optische Element (30) und ein zweites optisches Element (30) umfasst, wobei im Aufnahmebereich (24) der ersten Halterung (20) das erste optische Element (30) und im Aufnahmebereich (24) der zweiten Halterung (21) das zweite optische Element (30) aufgenommen sind, wobei das optische System (10) derart eingerichtet ist, dass entlang des ersten Linsensystemkanals (51) geführte Lichtbündel von dem ersten optischen Element (30) abgelenkt und entlang des zweiten Linsensystemkanals (52) geführte Lichtbündel von dem zweiten optischen Element (30) abgelenkt werden.

9. Optisches System (10) nach Anspruch 8, **dadurch gekennzeichnet, dass** die erste Halterung (20) und die zweite Halterung (21) symmetrisch zueinander im optischen System (10) angeordnet sind, wobei insbesondere die erste Halterung (20) achsensymmetrisch zur zweiten Halterung (21) in Bezug auf eine Längsachse (56) des optischen Systems (10) angeordnet sind, wobei die Längsachse (56) mittig zwischen der ersten optischen Achse (53) und der zweiten optischen Achse (54) verläuft.

10. Endoskop (2), insbesondere Stereo-Videoendoskop, umfassend ein optisches System (10) nach einem der Ansprüche 6 bis 9.

11. Verfahren zum Herstellen einer Halterung (20, 21) für ein optisches System (10) eines Endoskops (2) mit den folgenden, nacheinander auszuführenden Verfahrensschritten:
- Bereitstellen eines Rohlings (22) der Halterung (20, 21) aus einem Grundmaterial,
- Beschichten des Rohlings (22) mit einer Beschichtung, wobei die Beschichtung eine bessere Lötbarkeit als das Grundmaterial aufweist, wobei auf dem beschichteten Rohling (22) ein Fixierbereich (26, 27) zum Fixieren im optischen System (10) mittels einer Lötverbindung vorgesehen wird, **gekennzeichnet durch** den folgenden Verfahrensschritt:
- Bearbeiten eines Aufnahmebereichs (24) des beschichteten Rohlings (22) mit einem Laser (61), wobei der Aufnahmebereich (24) zur Aufnahme eines optischen Elements (30) eingerichtet und separat vom Fixierbereich (26, 27) ist, und wobei durch die Laserbearbeitung die Beschichtung (23) im Aufnahmebereich (24) abgetragen und die Oberfläche des Aufnahmebereichs (24) geschwärzt wird.

12. Verfahren nach Anspruch 11, **dadurch gekennzeichnet, dass** die Laserbearbeitung des Aufnahmebereichs (24) mit einem Pikosekundenlaser oder einem Femtosekundenlaser erfolgt.

13. Verfahren nach Anspruch 11 oder 12, **dadurch gekennzeichnet, dass** das Abtragen der Beschichtung (23) im Aufnahmebereich (24) und das Schwärzen des Aufnahmebereichs (24) mit einer einzigen Laserquelle (60) erfolgt, wobei insbesondere das Abtragen der Beschichtung (23) im Aufnahmebereich (24) und das Schwärzen des Aufnahmebereichs (24) in einem einzigen Arbeitsschritt durchgeführt werden.

## Claims

1. A holder (20, 21) for an optical system (10) of an endoscope (2), comprising a take-up region (24), configured to receive an optical element (30), and at least one fixing region (26, 27), which is configured to fix the holder (20, 21) in the optical system (10) by means of a soldered connection and the fixing region (26, 27) is separate from the take-up region (24), wherein the material of the holder (20, 21) is a base material that is provided with a coating (23), a material of the coating (23) having better solderability than the base material, **characterized in that** the coating (23) in the take-up region (24) being removed by means of laser-assisted surface treatment and the surface of the take-up region (24) being blackened by means of laser-assisted surface treatment.

2. The holder (20, 21) according to Claim 1, **characterized in that** the base material is a steel alloy, in particular a chrome steel.

3. The holder (20, 21) according to Claim 1 or 2, **characterized in that** the coating (23) in the take-up region (24) is removed by means of laser-assisted surface treatment with an picosecond or femtosecond-pulse laser and the surface of the take-up region (24) is blackened by means of laser-assisted surface treatment with an picosecond or femtosecond-pulse laser.

4. The holder (20, 21) according to any one of Claims 1 to 3, **characterized in that** the optical element (30), which the take-up region (24) is configured to receive, is an optical deflection prism.

5. The holder (20, 21) according to Claim 4, **characterized in that** the holder (20, 21) is configured to receive an image sensor (40) in such a manner that a light-sensitive sensor surface (41) of the received image sensor (40) extends parallel to a direction of incident light (42), wherein light bundles incident along the direction of incident light (42) into the optical deflection prism are deflected in the direction of the received image sensor (40) by means of the optical deflection prism.

6. An optical system (10) for an endoscope (2), comprising at least one holder (20, 21) according to any one of Claims 1 to 5 and at least one optical element (30), wherein the optical element (30) is received in the take-up region (24) of the holder (20, 21) and the holder (20, 21) is fixed in the optical system (10) by means of at least a first and a second soldered connection, wherein the first and second soldered connection connects the fixing region (26, 27) of the holder (20, 21) to a first or second fixing element (15, 16) of the optical system (10).

7. The optical system (10) according to Claim 6, **characterized in that** the holder (20, 21) has a first fixing region (26) and a second fixing region (27), wherein the first fixing region (26) is connected by means of the first soldered connection to the first fixing element (15) and the second fixing region (27) is connected by means of the second soldered connection to the second fixing element (16).

8. The optical system (10) according to Claim 6 or 7, **characterized in that** the optical system (10) is configured for use in a stereo-video endoscope, wherein the optical system (10) comprises a first lens system channel (51) having a first optical axis (53) and a second lens system channel (52) having a second optical axis (54), wherein the first optical axis (53) runs parallel to the second optical axis (54), and wherein the at least one holder (20, 21) of the optical system (10) comprises a first holder (20) according to any of claims 1 to 5 and a second holder (21) according to any of claims 1 to 5, and the at least one optical element (30) of the optical system (10) comprises a first optical element (30) and a second optical element (30), wherein the first optical element (30) is received in the take-up region (24) of the first holder (20) and the second optical element (30) is received in the take-up region (24) of the second holder (21), wherein the optical system (10) is configured **in that** light bundles guided along the first lens system channel (51) are diverted by the first optical element (30) and light bundles guided along the second lens system channel (52) are diverted by the second optical element (30).

9. The optical system (10) according to Claim 8, **characterized in that** the first holder (20) and the second holder (21) are arranged symmetrically to one another in the optical system (10), wherein the first holder (20) is in particular arranged axisymmetrically to the second holder (21) with respect to a longitudinal axis (56) of the optical system (10), wherein the longitudinal axis (56) runs centrally between the first optical axis (53) and the second optical axis (54).

10. An endoscope (2), in particular a stereo-video endoscope, comprising an optical system (10) according to any one of Claims 6 to 9.

11. A method for manufacturing a holder (20, 21) for an optical system (10) of an endoscope (2), having the following method steps which are to be carried out successively:
- providing a blank (22) of the holder (20, 21) made from a base material,
- coating the blank (22) with a coating, the coating having better solderability than the base material, wherein a fixing region (26, 27) for fixing in the optical system (10) is provided on the coated blank (22) by means of a soldered connection, **characterized by** the following step:
- treating a take-up region (24) of the coated blank (22) with a laser (61), wherein the take-up region (24) is configured to receive an optical element (30) and is separate from the fixing region (26, 27), and wherein the coating (23) in the take-up region (24) is removed and the surface of the take-up region (24) is blackened by the laser treatment.

12. The method according to Claim 11, **characterized in that** the laser treatment of the take-up region (24) is effected with an ultrashort-pulse laser.

13. The method according to Claim 11 or 12, **characterized in that** the removal of the coating (23) in the take-up region (24) and the blackening of the take-up region (24) are effected with a single laser source (60), wherein the removal of the coating (23) in the take-up region (24) and the blackening of the take-up region (24) are in particular performed in a single work step.

## Revendications

1. Support (20, 21) pour un système optique (10) d'un endoscope (2), comprenant une zone de réception (24) qui est conçue pour recevoir un élément optique (30), et au moins une zone de fixation (26, 27) qui est conçue pour fixer le support (20, 21) dans le système optique (10) au moyen d'une liaison par brasage et qui est séparée de la zone de réception (24), le matériau du support (20, 21) étant un matériau de base qui est pourvu d'un revêtement (23), un matériau du revêtement (23) présentant une meilleure aptitude au brasage que le matériau de base, **caractérisé en ce qu'**au moyen d'un traitement de surface au laser, le revêtement (23) est enlevé dans la zone de réception (24) et la surface de la zone de réception (24) est noircie au moyen du traitement de surface au laser.

2. Support (20, 21) selon la revendication 1, **caractérisé en ce que** le matériau de base est un alliage d'acier, en particulier un acier au chrome.

3. Support (20, 21) selon la revendication 1 ou la revendication 2, **caractérisé en ce que** le revêtement (23) dans la zone de réception (24) est enlevé au moyen d'un traitement de surface au laser avec un laser picoseconde ou un laser femtoseconde et la surface de la zone de réception (24) est noircie au moyen du traitement de surface au laser avec le laser picoseconde ou le laser femtoseconde.

4. Support (20, 21) selon l'une des revendications 1 à 3, **caractérisé en ce que** l'élément optique (30), pour la réception duquel la zone de réception (24) est aménagée, est un prisme de déviation optique.

5. Support (20, 21) selon la revendication 4, **caractérisé en ce que** le support (20, 21) est agencé pour recevoir un capteur d'image (40) de telle sorte qu'une surface de capteur photosensible (41) du capteur d'image (40) reçu s'étend parallèlement à une direction d'incidence (42), des faisceaux lumineux incidents dans le prisme optique de déviation le long de la direction d'incidence (42) étant déviés en direction du capteur d'image (40) reçu au moyen du prisme optique de déviation.

6. Système optique (10) pour un endoscope (2), comprenant au moins un support (20, 21) selon l'une des revendications 1 à 5 et au moins un élément optique (30), l'élément optique (30) étant logé dans la zone de réception (24) du support (20, 21) et le support (20, 21) est fixé dans le système optique (10) au moyen d'au moins une première ou une deuxième liaison soudée, la première ou la deuxième liaison soudée reliant la zone de fixation (26, 27) du support (20, 21) à un premier ou un deuxième élément de fixation (15, 16) du système optique (10).

7. Système optique (10) selon la revendication 6, **caractérisé en ce que** le support (20, 21) comprend une première zone de fixation (26) et une deuxième zone de fixation (27), la première zone de fixation (26) étant reliée au premier élément de fixation (15) au moyen de la première liaison soudée et la deuxième zone de fixation (27) étant reliée au deuxième élément de fixation (16) au moyen de la deuxième liaison soudée.

8. Le système optique (10) selon la revendication 6 ou la revendication 7, **caractérisé en ce que** le système optique (10) est conçu pour être utilisé dans un vidéo-endoscope stéréo, le système optique (10) comprenant un premier canal de système de lentilles (51) avec un premier axe optique (53) et un deuxième canal de système de lentilles (52) avec un deuxième axe optique (54), le premier axe optique (53) étant parallèle au deuxième axe optique (54), et dans lequel ledit au moins un support (20, 21) du système optique (10) comprend un premier support (20) selon l'une des revendications 1 à 5 et un deuxième support (21) selon l'une des revendications 1 à 5, et ledit au moins un élément optique (30) du système optique (10) comprend un premier élément optique (30) et un deuxième élément optique (30), le premier élément optique (30) étant reçu dans la zone de réception (24) du premier support (20) et le deuxième élément optique (30) étant reçu dans la zone de réception (24) du deuxième support (21), le système optique (10) étant agencé de telle sorte que des faisceaux lumineux guidés le long du premier canal du système de lentilles (51) soient déviés par le premier élément optique (30) et que des faisceaux lumineux guidés le long du deuxième canal du système de lentilles (52) soient déviés par le deuxième élément optique (30).

9. Système optique (10) selon la revendication 8, **caractérisé en ce que** le premier support (20) et le deuxième support (21) sont disposés symétriquement l'un par rapport à l'autre dans le système optique (10), le premier support (20) étant notamment agencé de façon axialement symétrique par rapport au deuxième support (21) par rapport à un axe longitudinal (56) du système optique (10), l'axe longitudinal (56) s'étendant de manière centrée entre le premier axe optique (53) et le deuxième axe optique (54).

10. Endoscope (2), notamment vidéo-endoscope stéréoscopique, comprenant un système optique (10) selon l'une quelconque des revendications 6 à 9.

11. Procédé de fabrication d'un support (20, 21) pour un système optique (10) d'un endoscope (2), comprenant les étapes de procédé suivantes, à exécuter successivement :
- la préparation d'une ébauche (22) du support (20, 21) à partir d'un matériau de base,
- le revêtement de l'ébauche (22) avec un revêtement, le revêtement présentant une meilleure aptitude au brasage que le matériau de base, une zone de fixation (26, 27) étant prévue sur l'ébauche revêtue (22) pour la fixation dans le système optique (10) au moyen d'une liaison par brasage, **caractérisé par** l'étape de procédé suivante :
- traitement d'une zone de réception (24) de l'ébauche revêtue (22) avec un laser (61), la zone de réception (24) étant aménagée pour recevoir un élément optique (30) et étant séparée de la zone de fixation (26, 27), et le traitement au laser enlevant le revêtement (23) dans la zone de réception (24) et noircissant la surface de la zone de réception (24).

12. Procédé selon la revendication 11, **caractérisé en ce que** le traitement au laser de la zone de réception (24) est effectué avec un laser picoseconde ou un laser femtoseconde.

13. Procédé selon la revendication 11 ou la revendication 12, **caractérisé en ce que** l'enlèvement du revêtement (23) dans la zone de réception (24) et le noircissement de la zone de réception (24) sont effectués avec une seule source laser (60), en particulier l'enlèvement du revêtement (23) dans la zone de réception (24) et le noircissement de la zone de réception (24) étant effectués en une seule étape de travail.
